# EUROPEAN PATENT SPECIFICATION

(11) **EP 0 882 028 B1**
(45) Date of publication and mention of the grant of the patent: **16.01.2002**
(21) Application number: 97901629.2
(22) Date of filing: 04.02.1997
(51) Int. Cl.: C07D 261/08, C07D 261/18

(54) **NEW INTERMEDIATES TO PESTICIDES**
ZWISCHENPRODUKTE FÜR PESTIZIDE
NOUVEAUX INTERMEDIAIRES DE PREPARATION DE PESTICIDES

(30) Priority: 16.02.1996 EP 96301070
(43) Date of publication of application: 09.12.1998
(73) Proprietor: AVENTIS CROPSCIENCE S.A., 69009 Lyon (FR)
(72) Inventor: PEVERE, Virginie, F-69008 Lyon (FR)
(74) Representative: Tetaz, Franck
(86) International application number: EP9700491
(87) International publication number: WO9730037

(56) References cited:
- EP-A- 0 487 357
- EP-A- 0 560 482
- EP-A- 0 609 798
- JOURNAL OF MOLECULAR CATALYSIS, vol. 54, 1989, THE NETHERLANDS, pages 73-80, XP000196064 A. C. VERONESE ET AL.: "Metal-catalysed Reaction of beta-Dicarbonyls with ethyl cynoformate: A new approach to the synthesis of unsaturated amino acis derivatives" cited in the application
- J. CHEM. SOC. PERKIN TRANS. I,, 1994, LONDON, pages 1779-1785, XP002010513 AUGUSTO C. VERONESE ET AL.: "Reactions of beta-Dicarbonyl Compounds with Acyl Cynides Catalysed or promoted by metal Centre in the Homogeneous phase"
- PAOLO GRÜNANGER, PAOLA VITA-FINZI: "Isoxazoles Part one", 1991, JOHN WILEY & SONS,

## Description

This invention relates to 2-(aminomethylidene)propane-1,3-dione derivatives, processes for their preparation, and their use as intermediates in the manufacture of herbicidally active compounds.

European patent Publication numbers 0487357, 0560482, 0580439, 0609797, 0609798, 0636622, 0682659 and International Patent Publication Number WO 95/16678 describe, *inter alia,* 3-ester-4-benzoylisoxazole herbicides. In particular, EP-A-0560482 describes the synthesis of ethyl 5-cyclopropyl-4-[3,4-dichloro-2-(methylsulphenyl)-benzoyl]isoxazole-3-carboxylate. Herbicidal 3-ester-4-heteroaroylisoxazoles are also known in the literature, for example in EP-A-0588357, EP-A-0636622 and WO96/25413. Each of these publications describe various processes for the preparation of such 3-ester-4-arylisoxazoles such as the reaction of a 1-cyclopropyl-3-aryl-propane-1,3,-dione with a chloro-oxime. It is however desirable to provide alternative routes for preparing such compounds

Certain 2-(aminomethylidene)-propane-1,3-dione derivatives are described in the literature. A. Veronese et al, J. Molecular Catalysis, Vol. 54 (1989) pp 73-80 describes the preparation of 2-(1'-amino-1'-ethoxycarbonylmethylidine)-1-phenylbutane-1,3-dione and Italian Patent No. 1200768 describes compounds of formula: 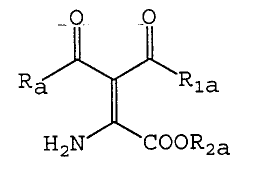 in which Rₐ and R₁ₐ, which may be the same or different, each represent C₁-C₄ alkyl, aryl or C₁-C₄ alkoxy, and R₂ₐ is C₁-C₄ alkyl or benzyl. These compounds are said to be useful as intermediates in the synthesis of, for example, pharmaceuticals.

It is an object of the present invention to provide a process for preparing herbicidally active compounds.

It is a further object of the invention to provide intermediates in the manufacture of herbicidally active compounds.

Invention to provide processes for preparing such intermediates and herbicides in high yield.

These and other objects of the invention will become apparent from the following description.

The present invention provides a process for preparing a compound of formula (I): 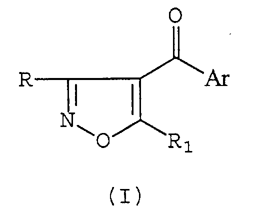 wherein:
R is -CO₂R³;
R¹ is lower alkyl or cycloalkyl having from three to seven ring carbon atoms, optionally substituted by lower alkyl or halogen;
Ar is:
   phenyl optionally substituted by one to five groups R² which may be the same or different;
   or is phenyl optionally substituted by from one to three groups R² which may be the same or different and wherein two additional substituents on adjacent positions of the phenyl ring, together with the two atoms to which they are attached, form a 5- to 7- membered ring which is optionally substituted by one or groups R¹¹ which may be the same or different;
   or is a group Het which is optionally substituted by one or more groups R², wherein Het represents a first heterocyclic ring containing from one to four heteroatoms in the ring selected from oxygen, nitrogen and sulphur, which is optionally fused with a benzene, or carbocyclic or second heterocyclic ring (which is optionally saturated or partially saturated) to form a bicyclic system, wherein the first heterocyclic ring of the group Het is attached to the carbonyl group in the 4-position of the isoxazole ring;
R² is halogen, lower alkyl, lower alkoxy, lower haloalkyl, lower haloalkoxy, -S(O)ₘR⁴, -(CR⁵R⁶)_{q}S(O)ₘR⁴, nitro, cyano, -N(R⁵)SO₂R⁸, -OSO₂R⁸, cycloalkyl having from three to seven ring carbon atoms, straight- or branched- chain alkenyl or alkynyl having up to six carbon atoms, -CO₂R⁷, -S(O)ₘR⁹, -O(CH₂)ᵣOR⁸, -NR⁵R⁶, -CONR⁵R⁶, -N(R⁵)C(=O)R¹⁰, -(CR⁵R⁶)_{q}N(R⁵)SO₂R⁸, -(CR⁵R⁶)_{q}N(R⁵)C(=O)R¹⁰, -(CR⁵R⁶)_{q}P(=O)R¹²R¹³, -(CR⁵R⁶)_{q}R¹⁴, -(CR⁵R⁶)_{q}R¹⁶, or a straight- or branched-chain alkyl containing up to six carbon atoms substituted by -OR⁵;
where R² is present on a heterocyclic or carbocyclic ring of the group Het, R² may also represent =O, =S, cyclic ketal or cyclic thioketal;
R³ is lower alkyl, lower haloalkyl, aryl or benzyl;
R⁴ is lower alkyl, or phenyl optionally substituted by from one to five groups selected from lower alkyl, lower haloalkyl, halogen and -S-alkyl;
R⁵ and R⁶ independently are hydrogen, lower alkyl or lower haloalkyl;
R⁷ is hydrogen or lower alkyl;
R⁸ is lower alkyl or lower haloalkyl;
R⁹ is lower haloalkyl, lower alkenyl or lower haloalkenyl;
R¹⁰ is R⁸, lower alkoxy or lower alkylthio;
R¹¹ is R² or =O, =S, lower alkylimino, cyclic ketal or cyclic thioketal;
R¹² and R¹³ are lower alkyl, hydroxy, lower alkoxy or lower haloalkoxy;
R¹⁴ is a 5-membered heteroaromatic ring of formula 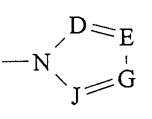
   in which D, E, G and J independently represent -CR¹⁵- or a nitrogen atom, with at least one of D, E, G and J representing -CR¹⁵-;
   two adjacent groups D, E, G and J may together form a second phenyl or 5- to 7-membered heteroaromatic ring optionally substituted by one or more groups selected from halogen or lower alkyl, in which the 5- to 7-membered heterocyclic ring contains from one to four heteroatoms in the ring which may be the same or different selected from nitrogen, oxygen and sulphur;
R¹⁵ is hydrogen, halogen, lower alkyl, lower haloalkyl, nitro, cyano, -CO₂R⁷, -S(O)ₘR⁸, lower alkoxy, lower haloalkoxy or cyclopropyl;
R¹⁶ is phenyl optionally substituted by halogen, nitro, cyano, lower alkyl, lower alkoxy, lower haloalkyl, lower haloalkoxy, -S(O)ₘR⁸, - (CR⁵R⁶)S(O)ₘR⁸;
m is zero, one or two;
q is one or two; and
r is one, two or three;
characterised by the reaction of a compound of formula (II): 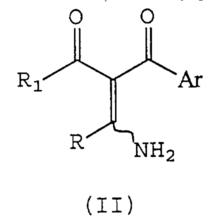 wherein R, R¹, and Ar are as defined above, with hydroxylamine or a salt thereof.

Hydroxylamine may be used in this reaction in the form of a salt, for example hydroxylamine sulfate or, hydroxylamine hydrochloride. Where the reaction is performed using a salt of hydroxylamine it is preferable, for reasons of speed of reaction, to include a base in the reaction mixture to liberate hydroxylamine and initiate the reaction. Examples of suitable bases include metal bases such as sodium acetate, carbonate or hydrogen carbonate; or organic bases, for example amines such as diisopropylamine and triethylamine. Where base is present, the ratio of base: hydroxylamine is generally from 0.01:1 1.5:1, preferably from 0.1:1 to 1.1:1.

The reaction is preferably performed in a solvent. Examples of suitable include:
aromatic, aliphatic and cycloaliphatic hydrocarbons and halogenated or non-halogenated hydrocarbons, for example dichloromethane, toluene, dichloroethane or chlorobenzene;
ether solvents such as tert butyl methyl ether (MTBE) and tetrahydrofuran.
an alcohol solvent such as ethanol and methanol;
or water.

It will be understood that the reaction may be performed in a mixture of two or more different solvents.

The reaction is preferably performed at a temperature of from 20°C to the boiling point of the solvent, preferably from 30 to 80°C

The molar ratio of the compound of formula (II): hydroxylamine is generally from 1:1 to 1:2, preferably from 1:1.01 to 1:1.2.

By the terms 'lower alkyl', 'lower alkenyl', 'lower alkoxy' or'lower alkylthio' is meant a straight- or branched- chain alkyl, alkenyl, alkoxy or alkylthio radical respectively, having from one to six carbon atoms.

By the term 'lower haloalkyl', 'lower haloalkenyl' or 'lower haloalkoxy' is meant a straight- or branched- chain alkyl, alkenyl or alkoxy radical respectively, having from one to six carbon atoms substituted by one or more halogens.

When a 5- to 7- membered ring forming part of the group Ar is present it may be carbocyclic or heterocyclic containing one or more heteroatoms, preferably selected from oxygen, sulphur and nitrogen (preferably from one to four), it being understood that a sulphur atom, where present, may be in the form of a group -SO- or -SO₂-, and may be aromatic, saturated or partially saturated.

Where R¹¹ represents cyclic ketal or cyclic thioketal preferably the ketal or thioketal ring contains 5 or 6 ring members.

Examples of the group Ar when it represents phenyl fused to a 5- to 7- membered ring include the following optionally substituted ring systems, wherein the substituents are as defined in claim 1 and corresponding dihydro compounds (where applicable):
benzo-1,2,3-thiadiazole, benzo[b]thiophene, benzo[b]furan, benzo[c]thiophene, benzo[c]furan, benzthiazole, 1,2-benzisothiazole, 2,1-benzisothiazole, 1,2-benzthiazin, 2,1-benzthiazin, 1,3-benzothiazine, 1,4-benzothiazine, benzimidazole, indazole, thiochroman, chroman, 2H-thiochromene, 2H-chromene, 4H-thiochromene, 4H-chromene, isothiochroman, isochroman, isothiochromene, isochromene, benzofurazan, 1,3-benzodithiole, 1,3-benzodioxole, 1,3-benzoxathiole, 1,4-benzodithiin, 1,4-benzoxathiin, 1,3-benzoxathiin, 3,1-benzoxathiin, 1,3-benzodithiin, thiochroman-4-one and saccharin.

Compounds of formula (I) above of this type are decribed in EP-A-0636622.

When a group Het is present preferably the first heterocyclic ring contains from 4 to 7 ring atoms, and the carbocyclic or second heterocyclic ring contains from 4 to 7 ring atoms. Het may be aromatic or non-aromatic. Examples of the ring system Het include:
thienyl, furyl, pyrrolyl and their benzo-fused analogues;
oxazinyl, thiazinyl, pyrazinyl, pyrimidinyl, pyridazinyl and their benzo-fused analogues;
thiazolyl, oxazolyl, imidazolyl and their benzo-fused analogues;
pyrazolyl, isoxazolyl, isothiazolyl and their benzo-fused analogues;
oxadiazolyl, thiadiazolyl, triazolyl and, where appropriate, their benzo-fused analogues;
pyridyl, pyranyl, thiinyl and their benzo-fused analogues;
oxadiazinyl, thiadiazinyl, triazinyl and, where appropriate, their benzo-fused analogues;
tetrazolyl, piperidinyl, morpholinyl and piperazinyl. Compound of formula (I) in which Ar is Het are described in EP-A-0588357.

Preferably the compound of formula (I) is a compound of formula (Ia) : 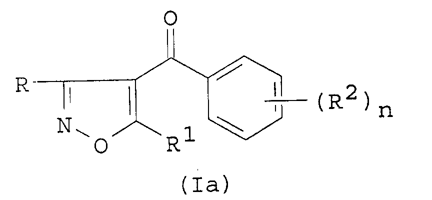
wherein R and R¹ are as defined above;
R² is halogen, lower alkyl, lower alkoxy, lower haloalkyl, lower haloalkoxy, -S(O)ₘR⁴, -(CR⁵R⁶)_{q}S(O)ₘR⁴, nitro, cyano, -N(R⁷)SO₂R⁸, -OSO₂R⁸, cycloalkyl having from three to seven ring carbon atoms; or straight- or branched-chain alkenyl or alkynyl having up to six carbon atoms;
R³ is lower alkyl, lower haloalkyl, aryl or benzyl;
n is an integer from one to five;
R⁴ is lower alkyl, or phenyl optionally substituted by from one to five groups selected from lower alkyl, lower haloalkyl, halogen and -S-alkyl;
R⁵ and R⁶ independently are hydrogen, lower alkyl or lower haloalkyl;
R⁷ is hydrogen or lower alkyl;
R⁸ is lower alkyl or lower haloalkyl;
m is zero, one or two; and q is one or two;
which is prepared by the reaction of a compound of formula (IIa): 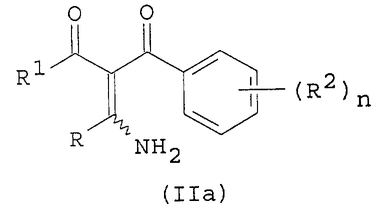 wherein R, R¹, R² and n are as defined above, with hydroxylamine or a salt thereof.

Compounds of formula (II) and (IIa) above are novel and therefore constitute a further feature of the present invention.

Compounds of formula (II) and (IIa) above in which R¹ is cycloalkyl having from three to seven ring carbon atoms, optionally substituted by lower alkyl or halogen are preferred. More preferably, R¹ is 1-methylcyclopropyl or, most preferably, cyclopropyl.

In formula (IIa) above, preferably one group R² is in the ortho-position of the phenyl ring.

R² is preferably selected from lower alkyl, lower haloalkyl, halogen and -S(O)ₘR⁴. More preferably R² is selected from -CF₃, halogen and -S(O)ₘR⁴.

In formula (IIa) above preferred groups (R²)ₙ include 2-SCH₃-3-Cl-4-Cl, 2-SOCH₃-3-Cl-4-Cl, 2-SCH₃-4-CF₃, 2-SO₂CH₃-4-CF₃ and 2-SO₂CH₃-4-halogen (preferably Cl or Br).
Compounds of formula (IIa) in which (R²)ₙ is 2-SCH₃-3-Cl-4-Cl or 2-SCH₃-4-CF₃ are particularly preferred.

Preferably R³ is lower alkyl, especially ethyl.

In formula (IIa) above preferably n is two or three.

The following examples illustrate the invention. In the description that follows "mM" means millimoles, and "m.p." means melting point.

### Example 1

### Preparation of ethyl 5-cyclopropyl-4-(3,4-dichloro-2-methylsulphenylbenzoyl)isoxazole-3-carboxylate.

1-Cyclopropyl-3-(3,4-dichloro-2-methylsulphenyl-phenyl)propane-1,3,-dione (9.087 g, 30mM) is charged to a 100ml reactor, followed by toluene (10 ml), ethyl cyanoformate (3.11 ml, 31.5mM) and zinc acetate dihydrate (132.5 mg, 0.6mM). The mixture is stirred at 80°C for 2.3 hours to give ethyl 2-amino-3-cyclopropylcarbonyl-4-oxo-4-(3,4-dichloro-2-methylsulphenyl-phenyl)but-2-enoate.

The reaction temperature is allowed to fall to 50°C. Hydroxylamine hydrochloride (2.296 g, 33 mM) and sodium acetate (0.494 g, 6mM) in ethanol (40 ml) are added and the mixture stirred at 50°C for 2.8 hours, by which time the reaction is complete. The reaction mixture is diluted by addition of toluene (15 ml) and the ethanol is eliminated by evaporation under reduced pressure at 45°C. Further toluene is added and the mixture again partially evaporated. The reaction is extracted at 50°C with water and the aqueous phases re-extracted with toluene. The toluene phases are evaporated to dryness under reduced pressure. The crude product (light orange solid) is dried to constant weight to give the title compound, (m.p. 79.5°C) in a yield of 11.84 g (theoretical 12g; a yield of 98.7%).

The preparation of 1-cyclopropyl-3-(3,4-dichloro-2-methylsulphenyl-phenyl)propane-1,3,-dione is described in EP-A-0560482, which also describes the synthesis of ethyl 5-cyclopropyl-4-(3,4-dichloro-2-methylsulphenylbenzoyl)isoxazole-3-carboxylate by treating the magnesium salt of 1-cyclopropyl-3-(3,4-dichloro-2-methylsulphenylphenyl)propane-1,3,-dione with ethyl chloro-oximidoacetate in dichloromethane (see Example 2 of EP-A-0560482). The reaction is reported as giving 2.19g of the title compound (5.47mM) from 2.0g of dione (6.60mM), i.e. a yield of 82.9% (cf. 98.7% in the present invention), which further illustrates the advantages of the present invention.

### Example 2

### Preparation of ethyl 2-amino-3-cyclopropylcarbonyl-4-oxo-4-(3,4-dichloro-2-methylsulphenyl-phenyl)but-2-enoate.

1-Cyclopropyl-3-(3,4-dichloro-2-methylsulphenyl-phenyl)propane-1,3-dione (1.82g, 6mM), ethyl cyanoacetate (0.66ml, 6.6mM) were stirred in 1,2-dichloroethane (5ml) for 1.5 hours at 50°C, then for 3 hours at 80°C. After cooling to ambient temperature, the solution was washed with water. The organic phase was evaporated under reduced pressure to give 2.5g of crude product, which was purified by column chromatography using a mixture of heptane and ethyl acetate to give a solid product which was crystallized in pentane to give 1.5g of the title product, m.p. 78°C.

### Example 3

### Preparation of ethyl 5-cyclopropyl-4-(3,4-dichloro-2-methylsulphenyl-benzoyl)isoxazole-3-carboxylate.

Ethyl 2-amino-3-cyclopropylcarbonyl-4-oxo-4-(2,4-dichloro-2-methylsulphenyl-phenyl)but-2-enoate (1.0g, 2.58mM) was dissolved in ethanol (7ml). Hydroxylamine hydrochloride (0.198g, 2.85mM) and sodium acetate (0.233g, 2.84mM) were added and the reaction stirred for one hour at 50°C. The reaction was cooled to room temperature and water added, followed by removal of the ethanol present under reduced pressure.
The reaction mixture was extracted with MTBE and the organic phase washed with water and evaporated under reduced pressure to give 0.82g of the title compound as a white solid.

### Example 4

### Preparation of ethyl 2-amino-3-cyclopropylcarbonyl-4-oxo-4-(2-methylsulphenyl-4-trifluoromethyl-phenyl)but-2-enoate.

1-Cyclopropyl-3-(2-methylsulphenyl-4-trifluoromethyl-phenyl)propane-1,3-dione (2.41g, 8mM)was charged to a 25 ml reactor followed by toluene (6 ml), zinc acetate dihydrate (35mg, 0.16 mM) and ethyl cyanoformate (860 microl, 8.7 mM). The mixture was stirred at 78°C for 4 hours. After cooling to ambient temperature , toluene (20 ml) was added to the mixture and the solution was wasched with brine. The organic phase is evaporated under reduced pressure to give 3.34 g of crude product as a viscous oil which was triturated with diethyl ether and pentane. After filtration of the insoluble material obtained, the solution was evaporated under reduced pressure to give 2.95 g of a dark viscous oil (Yield : 87.5% Assay : 95%).

### Example 5

### Preparation of ethyl 5-cyclopropyl-4-(2-methylsulphenyl-4-trifluoromethylbenzoyl)isoxazole-3-carboxylate.

Zinc acetate dihydrate (151 mg, 0.69 mM)was charged to a 50 ml reactor, followed by dichloromethane (14 ml),1-cyclopropyl-3-(2-methylsulphenyl-4-trifluoromethylphenyl)propane-1,3-dione (10 g, 33mM) and ethyl cyanoformate (3.7ml, 37.5mM). The mixture was stirred at 45°C for 6 hours to give ethyl 2-amino-3-cyclopropylcarbonyl-4-oxo-4-(2-methylsulphenyl-4-trifluoromethylphenyl)but-2-enoate.

Dichloromethane was distilled before adding ethanol (20ml).Hydroxylamine hydrochloride (2.53g, 36.38mM )in ethanol (5 ml)then sodium acetate (0.546g, 6.66mM) were added and the mixture stirred at 50°C for 4 hours. After cooling to ambient temperature, ethanol (10 ml) was added and the resulting yellow suspension was pourred onto water. The solid was filtered then dried to constant weight to give the title compound as a yellow solid (13g, m.p = 93 - 95°C) giving an overall yield of 93.4% (Assay : 95%).

## Claims

1. A process for preparing a compound of formula (I) 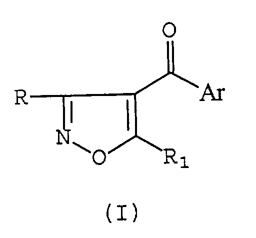 wherein
R is -CO₂R³;
R¹ is lower alkyl or cycloalkyl having from three to seven ring carbon atoms, optionally substituted by lower alkyl or halogen;
Ar is:
phenyl substituted by one to five groups R² which may be the same or different;
or is phenyl substituted by from one to three groups R² which may be the same or different and wherein two additional substituents on adjacent positions of the phenyl ring, together with the two atoms to which they are attached, form a 5- to 7- membered ring which is optionally substituted by one or groups R¹¹ which may be the same or different;
or is a group Het which is optionally substituted by one or more groups R², wherein Het represents a first heterocyclic ring containing from one to four heteroatoms in the ring selected from oxygen, nitrogen and sulphur, which is optionally fused with a benzene, or carbocyclic or second heterocyclic ring (which is optionally saturated or partially saturated) to form a bicyclic system, wherein the first heterocyclic ring of the group Het is attached to the carbonyl group in the 4-position of the isoxazole ring;
R² is halogen, lower alkyl, lower alkoxy, lower haloalkyl, lower haloalkoxy, -S(O)ₘR⁴, -(CR⁵R⁶)_{q}S(O)ₘR⁴, nitro, cyano, -N(R⁵)SO₂R⁸, -OSO₂R⁸, cycloalkyl having from three to seven ring carbon atoms, straight- or branched- chain alkenyl or alkynyl having up to six carbon atoms, -CO₂R⁷, -S(O)ₘR⁹, -O(CH₂)ᵣOR⁸, -NR⁵R⁶, -CONR⁵R⁶, -N(R⁵)C(=O)R¹⁰, -(CR⁵R⁶)_{q}N(R⁵)SO₂R⁸, -(CR⁵R⁶)_{q}N(R⁵)C(=O)R¹⁰, -(CR⁵R⁶)_{q}P(=O)R¹²R¹³, -(CR⁵R⁶)_{q}R¹⁴, -(CR⁵R⁶)_{q}R¹⁶, or a straight- or branched-chain alkyl containing up to six carbon atoms substituted by -OR⁵;
where R² is present on a heterocyclic or carbocyclic ring of the group Het, R² may also represent =O, =S, cyclic ketal or cyclic thioketal;
R³ is lower alkyl, lower haloalkyl, aryl or benzyl;
R⁴ is lower alkyl, or phenyl optionally substituted by from one to five groups selected from lower alkyl, lower haloalkyl, halogen and -S-alkyl;
R⁵ and R⁶ independently are hydrogen, lower alkyl or lower haloalkyl;
R⁷ is hydrogen or lower alkyl;
R⁸ is lower alkyl or lower haloalkyl;
R⁹ is lower haloalkyl, lower alkenyl or lower haloalkenyl;
R¹⁰ is R⁸, lower alkoxy or lower alkylthio;
R¹¹ is R² or =O, =S, lower alkylimino, cyclic ketal or cyclic thioketal;
R¹² and R¹³ are lower alkyl, hydroxy, lower alkoxy or lower haloalkoxy;
R¹⁴ is a 5-membered heteroaromatic ring of formula 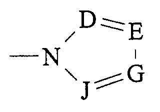
in which D, E, G and J independently represent -CR¹⁵- or a nitrogen atom, with at least one of D, E, G and J representing -CR¹⁵-;
two adjacent groups D, E, G and J may together form a second phenyl or 5- to 7-membered heteroaromatic ring optionally substituted by one or more groups selected from halogen or lower alkyl, in which the 5- to 7-membered heterocyclic ring contains from one to four heteroatoms in the ring which may be the same or different selected from nitrogen, oxygen and sulphur;
R¹⁵ is hydrogen, halogen, lower alkyl, lower haloalkyl, nitro, cyano, -CO₂R⁷, -S(O)ₘR⁸, lower alkoxy, lower haloalkoxy or cyclopropyl;
R¹⁶ is phenyl optionally substituted by halogen, nitro, cyano, lower alkyl, lower alkoxy, lower haloalkyl, lower haloalkoxy, -S(O)ₘR⁸, -(CR⁵R⁶)S(O)ₘR⁸ ;
m is zero, one or two;
q is one or two; and
r is one, two or three;
'lower alkyl', 'lower alkoxy' and 'lower alkylthio' mean straight- or branched- chain alkyl, alkoxy or alkylthio radical respectively, having from one to six carbon atoms; "lower alkenyl" means straight or branched-chain alkenyl radical having up to six carbon atoms.
'lower haloalkyl', 'lower haloalkenyl' or 'lower haloalkoxy' mean a straight- or branched-chain alkyl, alkenyl or alkoxy radical respectively, having from one to six carbon atoms substituted by one or more halogens;
lower haloalkenyl means a straight or branched chain alkenyl radical having up to six carbon atoms substituted by one or more halogens
**characterised by** the reaction of a compound of formula (II): 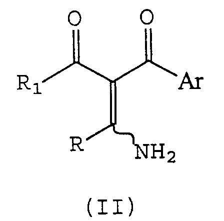 wherein R, R¹ and Ar are as defined above, with hydroxylamine or a salt thereof.

2. A process according to claim 1 for preparing a compound of formula (Ia): 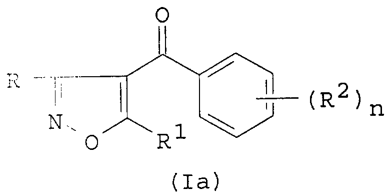
R and R¹ are as defined in claim 1 and
R² is halogen, lower alkyl, lower alkoxy, lower haloalkyl, lower haloalkoxy, -S(O)ₘR⁴, -(CR⁵R⁶)_{q}S(O)ₘR⁴, nitro, cyano, -N(R⁷)SO₂R⁸, -OSO₂R⁸, cycloalkyl having from three to seven ring carbon atoms; or straight- or branched-chain alkenyl or alkynyl having up to six carbon atoms;
R³ is lower alkyl, lower haloalkyl, aryl or benzyl ;
n is an integer from one to five;
R⁴ is lower alkyl, or phenyl optionally substituted by from one to five groups selected from lower alkyl, lower haloalkyl, halogen and -S-alkyl;
R⁵ and R⁶ independently are hydrogen, lower alkyl or lower haloalkyl;
R⁷ is hydrogen or lower alkyl;
R⁸ is lower alkyl or lower haloalkyl;
m is zero, one or two; and q is one or two; terms having the expression "lower" are as defined in claim 1
**characterised by** the reaction of a compound of formula (IIa): 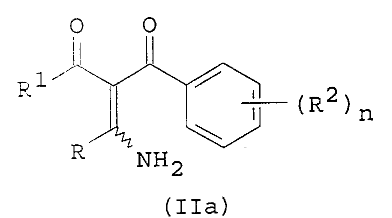 wherein R, R¹, R² and n are as defined above, with hydroxylamine or a salt thereof.

3. A process according to claim 1 or 2, **characterised in that** the reaction is performed at a temperature of from 20°C to the boiling point of the solvent, preferably from 30 to 80°C.

4. A process according to claim 1, 2 or 3, **characterised in that** the molar ratio of the compound of formula (II) : hydroxylamine is generally from 1:1 to 1:2, preferably from 1:1.01 to 1:1.2.

5. A process according to any one of claims 1 to 4, **characterised in that** the reaction is performed in the presence of a base.

6. A compound of formula (II): 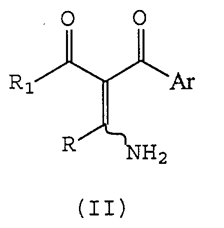 wherein R, R¹ and Ar are as defined in claim 1.

7. A compound according to claim 6 of formula (IIa): 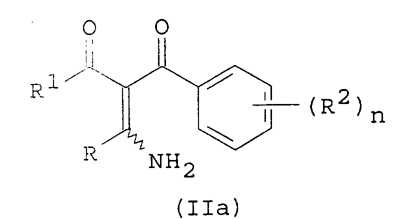 wherein R, R¹, R² and n are as defined in claim 2.

8. A compound according to claim 6 or 7 in which R¹ is cycloalkyl having from three to seven ring carbon atoms, optionally substituted by lower alkyl or halogen.

9. A compound according to claim 9 in which R¹ is 1-methylcyclopropyl or cyclopropyl.

10. The use of a compound of formula (II) as defined in claim 6 as an intermediate in the synthesis of a herbicide of formula (I) as defined in claim 1.

## Patentansprüche

1. Verfahren zur Herstellung einer Verbindung der Formel (I): 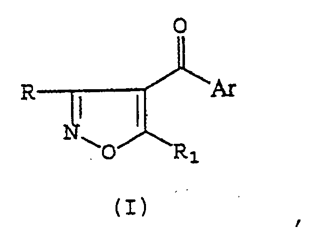 worin:
R für -CO₂R³ steht;
R¹ Niederalkyl oder Cycloalkyl mit drei bis sieben Ringkohlenstoffatomen bedeutet, gegebenenfalls substituiert durch Niederalkyl oder Halogen;
Ar für
Phenyl, das gegebenenfalls durch eine bis fünf Gruppen R² substituiert ist, die gleich oder verschieden sein können;
oder für Phenyl steht, das gegebenenfalls durch eine bis drei Gruppen R² substituiert ist, die gleich oder verschieden sein können und worin zwei weitere Substituenten an benachbarten Positionen des Phenylringes, zusammen mit den beiden Atomen, an die sie gebunden sind, einen 5- bis 7-gliedrigen Ring ausbilden, der gegebenenfalls durch eine oder durch Gruppen R¹¹ substituiert ist, die gleich oder verschieden sein können;
oder für eine Gruppe Het steht, die gegebenenfalls durch eine oder mehrere Gruppen R² substituiert ist, wobei Het einen ersten heterocyclischen Ring bedeutet, der ein bis vier Heteroatome im Ring enthält, ausgewählt unter Sauerstoff, Stickstoff und Schwefel, der gegebenenfalls mit einem Benzolring oder einem carbocyclischen oder zweiten heterocyclischen Ring kondensiert ist (der gegebenenfalls gesättigt oder partiell gesättigt ist), unter Ausbildung eines bicyclischen Systems, worin der erste heterocyclische Ring der Gruppe Het an die Carbonylgruppe in der 4-Position des Isoxazolringes gebunden ist;
R² für Halogen, Niederalkyl, Niederalkoxy, Niederhalogenalkyl, Niederhalogenalkoxy, -S(O)ₘR⁴, -(CR⁵R⁶)_{q}S(O)ₘR⁴, Nitro, Cyano, -N(R⁵)SO₂R⁸, -OSO₂R⁸, Cycloalkyl mit 3 bis 7 Ringkohlenstoffatomen, gerad- oder verzweigtkettiges Alkenyl oder Alkinyl mit bis zu sechs Kohlenstoffatomen, -CO₂R⁷, -S(O)ₘR⁹, -O(CH₂)ᵣOR⁸, -NR⁵R⁶, -CONR⁵R⁶, -N(R⁵)C(=O)R¹⁰, -(CR⁵R⁶)_{q}N(R⁵)SO₂R⁸, -(CR⁵R⁶)_{q}N(R⁵)C(=O)R¹⁰, -(CR⁵R⁶)_{q}P(=O)R¹²R¹³, -(CR⁵R⁶)_{q}R¹⁴, -(CR⁵R⁶)_{q}R¹⁶ oder ein gerad- oder verzweigtkettiges Alkyl mit bis zu sechs Kohlenstoffatomen steht, das durch -OR⁵ substituiert ist;
wobei R², wenn es auf einem heterocyclischen oder carbocyclischen Ring der Gruppe Het vorliegt, auch =O, =S, cyclisches Ketal oder cyclisches Thioketal sein kann;
R³ für Niederalkyl, Niederhalogenalkyl, Aryl oder Benzyl steht;
R⁴ für Niederalkyl oder Phenyl steht, gegebenenfalls substituiert durch eine bis fünf Gruppen, ausgewählt unter Niederalkyl, Niederhalogenalkyl, Halogen und -S-Alkyl;
R⁵ und R⁶ unabhängig voneinander Wasserstoff, Niederalkyl oder Niederhalogenalkyl bedeuten;
R⁷ Wasserstoff oder Niederalkyl darstellt;
R⁸ Niederalkyl oder Niederhalogenalkyl bedeutet;
R⁹ für Niederhalogenalkyl, Niederalkenyl oder Niederhalogenalkenyl steht;
R¹⁰ die Bedeutung R⁸, Niederalkoxy oder Niederalkylthio hat;
R¹¹ für R² oder =O, =S, Niederalkylimino, cyclisches Ketal oder cyclisches Thioketal steht;
R¹² und R¹³ Niederalkyl, Hydroxy, Niederalkoxy oder Niederhalogenalkoxy sind;
R¹⁴ einen 5-gliedrigen heteroaromatischen Ring der Formel 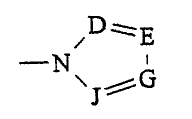 darstellt, worin D, E, G und J unabhängig voneinander für -CR¹⁵- oder ein Stickstoffatom stehen, wobei wenigstens eines der Symbole D, E, G und J für -CR¹⁵- steht;
zwei benachbarte Gruppen D, E, G und J zusammen einen zweiten Phenylring oder einen 5- bis 7-gliedrigen heteroaromatischen Ring ausbilden können, der gegebenenfalls durch eine oder mehrere Gruppen, ausgewählt unter Halogen oder Niederalkyl, substituiert ist, wobei der 5- bis 7-gliedrige heterocyclische Ring ein bis vier Heteroatome im Ring enthält, die gleich oder verschieden sein können, ausgewählt unter Stickstoff, Sauerstoff und Schwefel;
R¹⁵ für Wasserstoff, Halogen, Niederalkyl, Niederhalogenalkyl, Nitro, Cyano, -CO₂R⁷, -S(O)ₘR⁸, Niederalkoxy, Niederhalogenalkoxy oder Cyclopropyl steht;
R¹⁶ für Phenyl steht, das gegebenenfalls durch Halogen, Nitro, Cyano, Niederalkyl, Niederalkoxy, Niederhalogenalkyl, Niederhalogenalkoxy, -S(O)ₘR⁸, -(CR⁵R⁶)S(O)ₘR⁸ substituiert ist;
m den Wert null, eins oder zwei hat;
q den Wert eins oder zwei hat; und
r den Wert eins, zwei oder drei aufweist; wobei die Ausdrücke "Niederalkyl", "Niederalkoxy" und "Niederalkylthio" geradkettige oder verzweigtkettige Alkyl-, Alkoxy- bzw. Alkylthioreste mit 1 bis 6 Kohlenstoffatomen bedeuten;
"Niederalkenyl" einen geradkettigen oder verzweigtkettigen Alkenylrest mit bis zu 6 Kohlenstoffatomen bedeutet;
"Niederhalogenalkyl", "Niederhalogenalkenyl" oder "Niederhalogenalkoxy" einen geradkettigen oder verzweigtkettigen Alkyl-, Alkenyl- bzw. Alkoxyrest mit 1 bis 6 Kohlenstoffatomen bedeuten, die durch ein oder mehrere Halogenatome substituiert sind;
"Niederhalogenalkenyl" einen geradkettigen oder verzweigtkettigen Alkenylrest mit bis zu 6 Kohlenstoffatomen bedeutet, der durch ein oder mehrere Halogenatome substituiert ist;
**gekennzeichnet durch** die Umsetzung einer Verbindung der Formel (II): 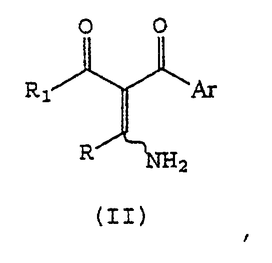 worin R, R¹ und Ar wie vorstehend definiert sind, mit Hydroxylamin oder einem Salz hievon.

2. Verfahren nach Anspruch 1 zur Herstellung einer Verbindung der Formel (Ia) : 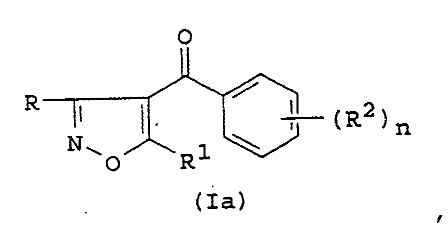 worin
R und R¹ wie in Anspruch 1 definiert sind und
R² für Halogen, Niederalkyl, Niederalkoxy, Niederhalogenalkyl, Niederhalogenalkoxy, -S(O)ₘR⁴, -(CR⁵R⁶)_{q}S(O)ₘR⁴, Nitro, Cyano, -N(R⁷)SO₂R⁸, -OSO₂R⁸, Cycloalkyl mit drei bis sieben Ringkohlenstoffatomen; oder für geradkettiges oder verzweigtkettiges Alkenyl oder Alkinyl mit bis zu sechs Kohlenstoffatomen steht;
R³ Niederalkyl, Niederhalogenalkyl, Aryl oder Benzyl ist;
n eine ganze Zahl von eins bis fünf bedeutet;
R⁴ für Niederalkyl oder Phenyl steht, das gegebenenfalls durch eine bis fünf Gruppen, ausgewählt unter Niederalkyl, Niederhalogenalkyl, Halogen und -S-Alkyl, substituiert ist;
R⁵ und R⁶ unabhängig voneinander Wasserstoff, Niederalkyl oder Niederhalogenalkyl bedeuten;
R⁷ für Wasserstoff oder Niederalkyl steht;
R⁸ für Niederalkyl oder Niederhalogenalkyl steht;
m den Wert null, eins oder zwei hat; und q den Wert eins oder zwei hat; wobei Begriffe mit dem Ausdruck "Nieder" wie im Anspruch 1 definiert sind,
**gekennzeichnet durch** die Umsetzung einer Verbindung der Formel (IIa): 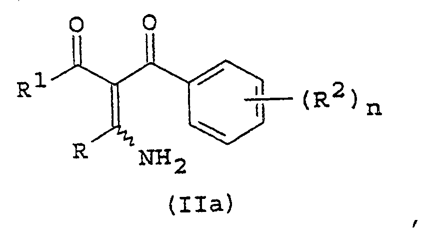 worin R, R¹, R² und n wie vorstehend definiert sind, mit Hydroxylamin oder einem Salz hievon.

3. Verfahren nach Anspruch 1 oder 2, **dadurch gekennzeichnet, daß** die Umsetzung bei einer Temperatur von 20°C bis zum Siedepunkt des Lösungsmittels ausgeführt wird, vorzugsweise bei 30 bis 80°C.

4. Verfahren nach Anspruch 1, 2 oder 3, **dadurch gekennzeichnet, daß** das Molverhältnis der Verbindung der Formel (II):Hydroxylamin generell von 1:1 bis 1:2 beträgt, vorzugsweise von 1:1,01 bis 1:1,2.

5. Verfahren nach einem der Ansprüche 1 bis 4, **dadurch gekennzeichnet, daß** die Umsetzung in Gegenwart einer Base vorgenommen wird.

6. Eine Verbindung der Formel (II): 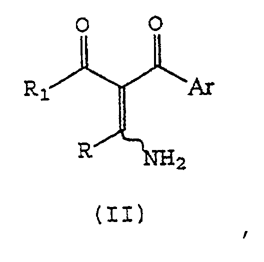 worin R, R¹ und Ar wie in Anspruch 1 definiert sind.

7. Verbindung nach Anspruch 6 mit der Formel (IIa) : 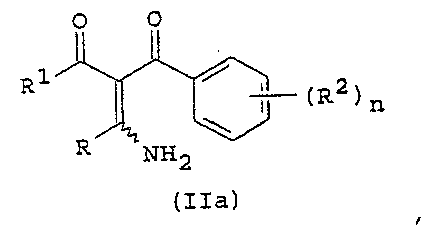 worin R, R¹, R² und n wie in Anspruch 2 definiert sind.

8. Verbindung nach Anspruch 6 oder 7, worin R¹ Cycloalkyl mit 3 bis 7 Ringkohlenstoffatomen bedeutet, das gegebenenfalls durch Niederalkyl oder Halogen substituiert ist.

9. Verbindung nach Anspruch 9, worin R¹ 1-Methylcyclopropyl oder Cyclopropyl darstellt.

10. Die Verwendung einer Verbindung der Formel (II) gemäß Definition in Anspruch 6 als Zwischenprodukt in der Synthese eines Herbizids der Formel (I) gemäß Definition in Anspruch 1.

## Revendications

1. Procédé de préparation d'un composé de formule (I) 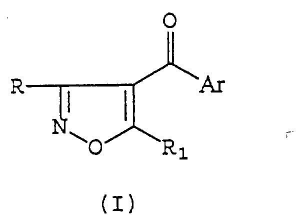 dans laquelle :
R est -CO₂R³ ;
R1 est un groupe alkyle inférieur ou un groupe cycloalkyle ayant trois à sept atomes de carbone cycliques, facultativement substitué par un groupe alkyle inférieur ou halogéno ;
Ar est :
un groupe phényle facultativement substitué par un à cinq groupes R² qui peuvent être identiques ou différents ;
ou est un groupe phényle facultativement substitué par un à trois groupes R² qui peuvent être identiques ou différents et où deux substituants supplémentaires liés à des positions adjacentes du noyau phényle forment, avec les deux atomes auquel ils sont fixés, un cycle penta- à heptagonal qui est facultativement substitué par un ou plusieurs groupes R¹¹ qui peuvent être identiques ou différents ;
ou est un groupe Het qui est facultativement substitué par un ou plusieurs groupes R², où Het représente un premier hétérocycle contenant un à quatre hétéroatomes cycliques choisis parmi l'oxygène, l'azote et le soufre, qui est facultativement condensé avec un noyau benzénique ou avec un carbocycle ou un second hétérocycle (qui est facultativement saturé ou partiellement saturé) pour former un système bicyclique, le premier hétérocycle du groupe Het étant fixé au groupe carbonyle à la position 4 du cycle d'isoxazole ;
R² est un halogène, un groupe alkyle inférieur, alcoxy inférieur, halogénoalkyle inférieur, halogénoalcoxy inférieur, -S(O)ₘR⁴, -(CR⁵R⁶)_{q}S(O)ₘR⁴, nitro, cyano, -N(R⁵)SO₂R⁸, -OSO₂R⁸, cycloalkyle ayant trois à sept atomes de carbone cycliques, alcényle ou alcynyle à chaîne linéaire ou ramifiée ayant jusqu'à six atomes de carbone, -CO₂R⁷, -S(O)ₘR⁹, -O(CH₂)ᵣOR⁸, -NR⁵R⁶, -CONR⁵R⁶, -N(R⁵)C(=O)R¹⁰, -(CR⁵R⁶)_{q}N(R⁵)SO₂R⁸, -(CR⁵R⁶)_{q}N(R⁵)C(=O)R¹⁰, -(CR⁵R⁶)_{q}P(=O)R¹²R¹³, -(CR⁵R⁶)_{q}R¹⁴, -(CR⁵R⁶)_{q}R¹⁶ ou un groupe alkyle à chaîne linéaire ou ramifiée contenant jusqu'à six atomes de carbone substitué par -OR⁵ ;
lorsque R² est présent sur un hétérocycle ou carbocycle du groupe Het, R² peut également représenter =O, =S, un cétal cyclique ou un thiocétal cyclique ;
R³ est un groupe alkyle inférieur, halogénoalkyle inférieur, aryle ou benzyle ;
R⁴ est un groupe alkyle inférieur ou un groupe phényle facultativement substitué par un à cinq groupes choisis parmi les groupes alkyle inférieur, halogénoalkyle inférieur, halogéno et -S-alkyle ;
R⁵ et R⁶ sont chacun indépendamment l'hydrogène, un groupe alkyle inférieur ou halogénoalkyle inférieur ;
R⁷ est l'hydrogène ou un groupe alkyle inférieur ;
R⁸ est un groupe alkyle inférieur ou halogénoalkyle inférieur ;
R⁹ est un groupe halogénoalkyle inférieur, alcényle inférieur ou halogénoalcényle inférieur ;
R¹⁰ est R⁸, un groupe alcoxy inférieur ou alkylthio inférieur ;
R¹¹ est R² ou =O, =S, un groupe (alkyle inférieur)imino, cétal cyclique ou thiocétal cyclique ;
R¹² et R¹³ sont des groupes alkyle inférieur, hydroxyle, alcoxy inférieur ou halogénoalcoxy inférieur ;
R¹⁴ est un noyau hétéroaromatique pentagonal de formule 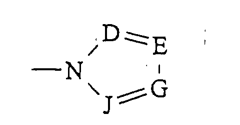
dans laquelle D, E, G et J représentent chacun indépendamment -CR¹⁵- ou un atome d'azote, l'un au moins de D, E, G et J représentant -CR¹⁵- ;
deux groupes adjacents D, E, G et J peuvent former ensemble un second noyau phényle ou hétéroaromatique penta- à heptagonal facultativement substitué par un ou plusieurs groupes choisis parmi les groupes halogéno ou alkyle inférieur, le noyau hétérocyclique penta- à heptagonal contenant un à quatre hétéroatomes cycliques qui peuvent être identiques ou différents et sont choisis parmi l'azote, l'oxygène et le soufre ;
R¹⁵ est l'hydrogène, un halogène, un groupe alkyle inférieur, halogénoalkyle inférieur, nitro, cyano, -CO₂R⁷, -S(O)ₘR⁸, alcoxy inférieur, halogénoalcoxy inférieur ou cyclopropyle ;
R¹⁶ est un groupe phényle facultativement substitué par un groupe halogéno, nitro, cyano, alkyle inférieur, alcoxy inférieur, halogénoalkyle inférieur, halogénoalcoxy inférieur, -S(O)ₘR⁸, -(CR⁵R⁶)S(O)ₘR⁸ ;
m est zéro, un ou deux ;
q est un ou deux ; et
r est un, deux ou trois ;
"alkyle inférieur", "alcoxy inférieur" et "alkylthio inférieur" désignent respectivement un groupe alkyle, alcoxy ou alkylthio à chaîne linéaire ou ramifiée ayant un à six atomes de carbone ; "alcényle inférieur" désigne un groupe alcényle à chaîne linéaire ou ramifiée ayant jusqu'à six atomes de carbone ;
"halogénoalkyle inférieur", "halogénoalcényle inférieur" et "halogénoalcoxy inférieur" désignent respectivement un groupe alkyle, alcényle ou alcoxy à chaîne linéaire ou ramifiée ayant un à six atomes de carbone, substitué par un ou plusieurs atomes d'halogène ; "halogénoalcényle inférieur désigne un groupe alcényle à chaîne linéaire ou ramifiée ayant jusqu'à six atomes de carbone, substitué par un ou plusieurs atomes d'halogène ;
**caractérisé par** la réaction d'un composé de formule (II) : 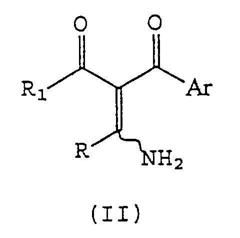 dans laquelle R, R¹ et Ar sont tels que définis ci-dessus,
avec l'hydroxylamine ou l'un de ses sels.

2. Procédé selon la revendication 1, pour la préparation d'un composé de formule (Ia) : 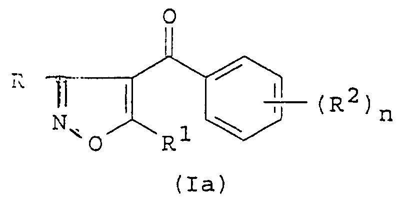
R et R¹ sont tels que définis dans la revendication 1 et
R² est un halogène, un groupe alkyle inférieur, alcoxy inférieur, halogénoalkyle inférieur, halogénoalcoxy inférieur, -S(O)ₘR⁴, -(CR⁵R⁶)_{q}S(O)ₘR⁴, nitro, cyano, -N(R⁷)SO₂R⁸, -OSO₂R⁸, cycloalkyle ayant trois à sept atomes de carbone cycliques ; ou alcényle ou alcynyle à chaîne linéaire ou ramifiée ayant jusqu'à six atomes de carbone ;
R³ est un groupe alkyle inférieur, halogénoalkyle inférieur, aryle ou benzyle ;
n est un nombre entier de un à cinq ;
R⁴ est un groupe alkyle inférieur ou un groupe phényle facultativement substitué par un à cinq groupes choisis parmi les groupes alkyle inférieur, halogénoalkyle inférieur, halogéno et -S-alkyle ;
R⁵ et R⁶ sont chacun indépendamment l'hydrogène, un groupe alkyle inférieur ou halogénoalkyle inférieur ;
R⁷ est l'hydrogène ou un groupe alkyle inférieur ;
R⁸ est un groupe alkyle inférieur ou halogénoalkyle inférieur ;
m est zéro, un ou deux ; et q est un ou deux ;
les expressions comportant le terme "inférieur" sont telles que définies dans la revendication 1,
**caractérisé par** la réaction d'un composé de formule (IIa) : 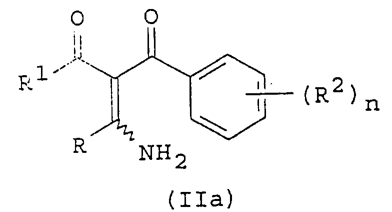 dans laquelle R, R¹, R² et n sont tels que définis ci-dessus,
avec l'hydroxylamine ou l'un de ses sels.

3. Procédé selon la revendication 1 ou 2, **caractérisé en ce que** la réaction est conduite à une température comprise entre 20°C et le point d'ébullition du solvant, de préférence entre 30 et 80°C.

4. Procédé selon la revendication 1, 2 ou 3, **caractérisé en ce que** le rapport molaire du composé de formule (II):hydroxylamine est généralement de 1:1 à 1:2, de préférence de 1:1,01 à 1:1,2.

5. Procédé selon l'une quelconque des revendications 1 à 4, **caractérisé en ce que** la réaction est conduite en présence d'une base.

6. Composé de formule (II) : 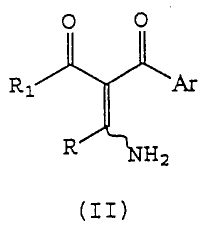 dans laquelle R, R¹ et Ar sont tels que définis dans la revendication 1.

7. Composé selon la revendication 6, de formule (IIa) : 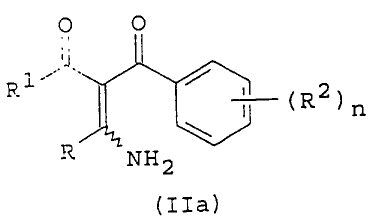 dans laquelle R, R¹, R² et n sont tels que définis dans la revendication 2.

8. Composé selon la revendication 6 ou 7, dans lequel R¹ est un groupe cycloalkyle ayant trois à sept atomes de carbone cycliques, facultativement substitué par un groupe alkyle inférieur ou halogéno.

9. Composé selon la revendication 9, dans lequel R¹ est un groupe 1-méthylcyclopropyle ou cyclopropyle.

10. Utilisation d'un composé de formule (II) tel que défini dans la revendication 6 comme intermédiaire dans la synthèse d'un herbicide de formule (I) tel que défini dans la revendication 1.
